# EUROPÄISCHE PATENTANMELDUNG

(11) **EP 3 009 420 A1**
(43) Veröffentlichungstag der Anmeldung: **20.04.2016**
(21) Anmeldenummer: 14189192.9
(22) Anmeldetag: 16.10.2014
(51) Int. Cl.: C07C 231/12

(54) **Verfahren zum Herstellen von Biphenylaminen aus Aniliden durch Rutheniumkatalyse**

(71) Anmelder: Bayer CropScience AG, 40789 Monheim am Rhein (DE); Georg-August-Universität Göttingen Stiftung Öffentlichen Rechts, 37073 Göttingen (DE)
(72) Erfinder: Himmler, Thomas, 51519 Odenthal (DE); Rodefeld, Lars, 51375 Leverkusen (DE); Hubrich, Jonathan, 37073 Göttingen (DE); Ackermann, Lutz, 37077 Göllingen (DE)
(74) Vertreter: BIP Patents

(57) **Zusammenfassung**

Die vorliegende Erfindung betrifft ein neues Verfahren zum Herstellen von substituierten Biphenylamiden der allgemeinen Formel (V) 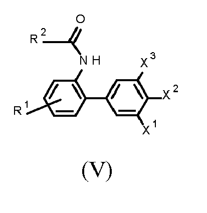 dadurch gekennzeichnet, dass man
Anilide der Formel (II) 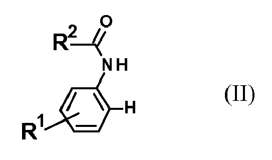 in einem von Tetrahydrofuran verschiedenen Lösungsmittel
mit einer Organoborverbindung der Formel (III) 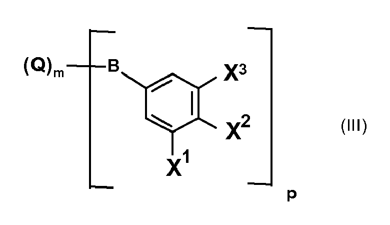 in Gegenwart eines Katalysatorsystems bestehend aus einem Rutheniumkatalysator, einem Aktivierungsmittel, einem Oxidationsmittel und einem Metalltriflat umsetzt.

## Beschreibung

Die vorliegende Erfindung betrifft ein neues Verfahren zum Herstellen von substituierten Biphenylamiden und in einer weiteren optionalen Stufe Biphenylaminen.

Biarylverbindungen, insbesondere Biphenylverbindungen, haben technische Bedeutung als Feinchemikalien, Zwischenprodukte für Pharmazeutika, optische Aufheller und Agrochemikalien.

Eine grundsätzlich mögliche Methode zur Herstellung von Biarylverbindungen ist in der übergangsmetallkatalysierten Kreuzkupplung unter doppelter C-H-Aktivierung zu sehen (siehe beispielsweise S. L. Buchwald et al, Org. Lett. 2008, 10(11), 2207-10; F. Glorius et al., Angew. Chem. Int. Ed. 2012, 51, 2247-51; WO 2014/019995). Diese Methoden ersparen zwar die Synthese beispielsweise einer Boronsäure als Ausgangsverbindung, haben aber schwerwiegende Nachteile. So werden üblicherweise teure Palladium- oder Rhodiumkomplexe als Katalysatoren eingesetzt. Weiterhin führt die allgemein geringe Reaktivität von C-H Bindungen häufig zu Selektivitätsproblemen (Funktionalisierung einer C-H Bindung in Gegenwart von anderen C-H Bindungen. Außerdem gibt es eine Konkurrenz zwischen Hetero- und Homokupplung.

Weiterhin ist bereits bekannt, dass sich Biphenyl-Derivate aus Phenylboronsäuren und Phenylhalogeniden durch eine Suzuki- oder Stille-Kupplung, d.h. durch eine palladiumkatalysierte Reaktion, herstellen lassen (vgl. z.B. WO 01/42223, WO 03/070705, WO 07/138089. WO 09/003650, WO 09/135598).

Weiterhin ist bekannt, dass man Biphenyl-Derivate erhält, indem man Aryl-Zink-Halogenide mit Arylhalogeniden umsetzt (Bull. Korean Chem. Soc. 2000, 21, 165-166).

Nachteilig bei diesen Verfahren sind die hohen Herstellkosten. Die übergangsmetallkatalysierten Kreuzkupplungen (z.B. nach Suzuki) erfordern relativ hohe Mengen an teuren Palladiumkatalysatoren oder aber (Bull. Korean Chem. Soc. 2000, 21, 165-166) die Verwendung von nahezu äquivalenten Mengen Zink, das als Abfall entsorgt werden muss. Zur Aktivierung des Zinks wird überdies das cancerogene Dibrommethan benötigt.

Weiterhin ist bekannt, dass man Biphenylderivate erhält, indem man Acetanilide mit aromatischen Boronsäuren in Gegenwart von Palladiumkatalysatoren, Kupfer(II)-triflat (Cu(OTf)₂) und Silberoxid (Ag₂O) umsetzt (Z.Shi et al., Angew.Chem. Int. Ed. 46 (2007) 5554-8). Auch hier sind die hohen Kosten für den Palladiumkataylsator von Nachteil.

Ebenfalls ist bekannt, dass man Biphenylderivate erhält, indem man Arylharnstoff Verbindungen mit aromatischen Boronsäuren in Gegenwart von Palladiumkatalysatoren und Benzochinon umsetzt (B.H.Lipshutz et al., J.Amer.Chem.Soc. 132 (2010) 4978-9). Wiederum sind die hohen Kosten für den Palladiumkataylsator von Nachteil.

Weiterhin ist bekannt, dass man Biphenylderivate erhält, indem man Acetanilide mit aromatischen Boronsäuren in Gegenwart von Ruthenium(II)-Komplexen, Silberhexafluoroantimonat (AgSbF₆), Cu(OTf)₂ und Ag₂O umsetzt (R.K.Chinnagolla und M.Jeganmohan, Chemical Communication, Januar 2014, accepted for publication).

Die Autoren geben jedoch an, daß als Lösungsmittel lediglich Tetrahydrofuran geeignet ist, während andere genannte technisch bevorzugte Lösungsmittel wie beispielsweise Methanol, Toluol oder Dimethylformamid, als völlig ineffektiv, d.h. im Sinne der Offenbarung ohne Umsatz beschrieben werden, so daß die Reaktion für den Fachmann nicht ausführbar und somit auch nicht offenbart ist.

Die Aufgabe der vorliegenden Erfindung bestand also in der Bereitstellung eines neuen Verfahrens, durch das Biphenylamine mit hoher Gesamtausbeute und hoher Reinheit ohne die Verwendung von teuren Palladiumkatalysatoren und unter technisch bevorzugten Reaktionsbedingungen, insbesondere mit technisch bevorzugten Lösunsgmitteln, erhalten werden können.

Gegenstand der vorliegenden Erfindung ist demnach ein Verfahren zur Herstellung von Biphenylamiden der allgemeinen Formel (V) und nachfolgend in einer optionalen zweiten Stufe Biphenylaminen der allgemeinen Formel (I) 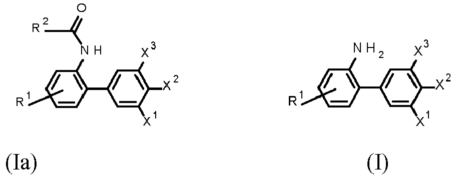 in welcher
- R¹: für Wasserstoff, Hydroxy, Fluor, Chlor, C₁-C₄-Alkyl, C₁-C₄-Alkoxy, C₁-C₄-Alkylthio oder C₁-C₄-Halogenalkyl steht,
- R²: für C₁-C₄-Alkyl, C₆-C₁₀-Aryl oder C₆-C₁₀-Aryl-CH₂- steht, und
- X¹: für Wasserstoff, C₁-C₄-Alkyl, C₁-C₄-Alkoxy, Fluor oder Chlor steht,
- X²: für Wasserstoff, C₁-C₄-Alkyl, C₁-C₄-Alkoxy, Fluor oder Chlor steht,
- X³: für Wasserstoff, C₁-C₄-Alkyl, C₁-C₄-Alkoxy, Fluor oder Chlor steht,
dadurch gekennzeichnet, dass man
(1) in einem ersten Schritt
Anilide der Formel (II) 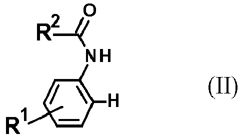 in welcher
- R¹ und R²: die oben angegebenen Bedeutungen haben,
in einem von Tetrahydrofuran (THF) verschiedenen Lösungsmittel
mit einer Organoborverbindung der Formel (III) 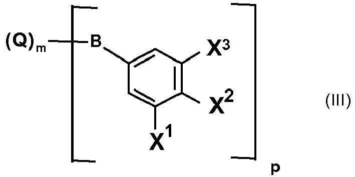 in welcher
X¹, X² und X³ die oben angegebenen Bedeutungen haben,
und die aus einer der folgenden Gruppen gewählt ist, bestehend aus
(I) Boronsäuren der Formel (III), in der
   Q für eine Hydroxygruppe steht,
   m für die Zahl 2 steht,
   p für die Zahl 1 steht,
   oder den Anhydriden, Dimeren oder Trimeren dieser Boronsäuren;
(II) Boronsäurederivaten der Formel (III), in der
   Q für F, Cl, Br, I, C₁-C₄-Alkyl, C₆-C₁₀-Aryl, C₁-C₄-Alkoxy oder C₆-C₁₀-Aryloxy steht, m für die Zahl 2 steht,
   p für die Zahl 1 steht;
(III) Borinsäuren der Formel (III), in der
   Q für OH, F, Cl, Br, I, C₁-C₄-Alkyl, C₆-C₁₀-Aryl, C₁-C₄-Alkoxy oder C₆-C₁₀-Aryloxy steht,
   m für die Zahl 1 steht,
   p für die Zahl 2 steht;
(IV) cyclische Boronsäureester der Formel (III), in der
   Q für einen C₂-C₃-Alkyldioxy-Rest steht, der zusammen mit dem Boratom, an das er gebunden ist, einen 5- oder 6-gliedrigen Ring bildet, der gegebenfalls durch einen oder mehrere C₁-C₄-Alkyl-Reste substituiert ist,
   m für die Zahl 2 steht,
   p für die Zahl 1 steht;
(V) Boronate der Formel (III), in der
   Q für OH, F, Cl, Br, I, C₁-C₄-Alkyl, C₆-C₁₀-Aryl, C₁-C₄-Alkoxy oder C₆-C₁₀-Aryloxy steht,
   m für die Zahl 3 steht,
   p für die Zahl 1 steht
   und die negative Ladung des Boronatanions durch ein Kation ausgeglichen wird, vorzugsweise durch ein Metallkation, wobei das Metall weiter bevorzugt ausgewählt ist aus den Metallen der 1. und 2. Hauptgruppe sowie Aluminium, Eisen und Kupfer.
(VI) Triarylborane der Formel (III), in der
   m für die Zahl 0 steht,
   p für die Zahl 3 steht;
(VII) Tetraarylborate der Formel (IV), in der
   m für die Zahl 0 steht,
   p für die Zahl 4 steht,
   und die negative Ladung des Tetraarylboratanions durch ein Kation ausgeglichen wird, vorzugsweise durch ein Metallkation, wobei das Metall weiter bevorzugt ausgewählt ist aus den Metallen der 1. und 2. Hauptgruppe sowie Aluminium, Eisen und Kupfer,
in Gegenwart eines Katalysatorsystems bestehend aus einem Rutheniumkatalysator, einem Aktivierungsmittel, einem Oxidationsmittel und einem Metalltriflat umsetzt, wobei das Metall vorzugsweise ausgewählt ist aus der Gruppe, die Li, Na, K, Mg, Ca, Mn, Fe, Co, Ni, Cu und Zn umfaßt, weiter bevorzugt aus der Gruppe die Fe und Ni umfaßt, und besonders bevorzugt Fe umfaßt. Vorzugsweise liegen Cu und Fe dabei in ihrer jeweils höchsten Oxidationsstufe vor, während alle anderen Metalle, ausgenommen der Alkalimetalle, vorzugsweise in der Oxidationsstufe +II vorliegen.

In einer optionalen zweiten Stufe können die so erhaltenen Anilide der Formel (V) 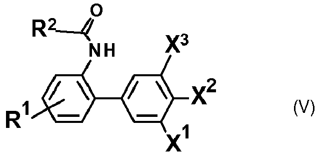 in welcher R¹, R² X¹, X² und X³ die oben angegebenen Bedeutungen haben,
nach bekannten Methoden der organischen Chemie sauer oder basisch verseift (Entfernung der Schutzgruppe [-C(=O)R²] am Stickstoff) werden.
Vorzugsweise wird die Schutzgruppe am Stickstoff in der zweiten Stufe entfernt.

In einer bevorzugten Ausführungsform werden Boronsäuren als Komponente der Formel (III) eingesetzt, wobei besonders bevorzugt Q=OH, m=2 und p=1 ist.

C₁-C₄-Alkyl umfaßt Methyl, Ethyl, Propyl und Isopropyl, Butyl, Isobutyl und tert.-Butyl und ist besonders bevorzugt Methyl.
C₁-C₄-Alkoxy umfaßt Methoxy, Ethoxy, Propoxy , Isopropoxy und Butoxy und ist besonders bevorzugt Methoxy.

In einer alternativen Ausführungsform besteht das Katalysatorsystems aus einem Rutheniumkatalysator, einem Aktivierungsmittel, einem Oxidationsmittel und einem Metallsulfat, wobei das Metall vorzugsweise ausgewählt ist aus der Gruppe, die Mn(II), Fe(III), Co(II), Ni(II), Cu(II), Zn(II) Mg(II), Ca(II) und Al(III) umfaßt, weiter bevorzugt aus der Gruppe die Fe(II) und Cu(II) umfaßt, und besonders bevorzugt Cu(II) umfaßt.
Der Vorteil dieser Ausführungsform besteht darin, daß bei nur wenig verringerter Ausbeute gegenüber den Triflaten die deutlich preisgünstigeren und somit wirtschaftlich interessanteren Sulfate verwendet werden können.

In einer weiteren alternativen Ausführungsform ist der Aktivator des Katalysatorsystems ausgewählt aus der Gruppe die Cu(I)-Oxid und Cu(II)-Oxid umfaßt.

Während sich die alternativen Ausführungsformen in der o.g. Art des Metallsalzes unterscheiden, gelten desweiteren für die übrigen Raktionsparameter und Reaktionspartner die Vorgaben der vorliegenden Beschreibung. Ausgenommen hiervon ist, daß bei Reaktionen ohne Metalltriflat auch THF als Lösungsmittel mit guten Ausbeuten verwendet werden kann.

Überraschenderweise lassen sich durch diese Reaktionssequenz die Biphenylamine der Formel (I) in guten Ausbeuten ohne die Verwendung von halogenierten Aniliden, ohne Verwendung teurer Palladiumkatalysatoren und unter technisch vorteilhaften Reaktionsbedingungen, insbesonders hinsichtlich der verwendeten Lösungsmittel, herstellen, die im Gegensatz zu Tetrahydrofuranen nicht zur Peroxidbildung neigen.

Verwendet man N-(4-Fluorphenyl)acetamid und Phenylboronsäure als Ausgangsstoffe, so kann das erfindungsgemäße Verfahren durch das folgenden Formelschema beispielhaft veranschaulicht werden.

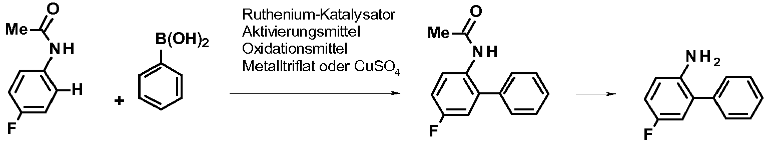

Verwendet man N-(4-Fluorphenyl)acetamid und beispielsweise Phenylborinsäure als Ausgangsstoffe, so kann des erfindungsgemäße Verfahren durch das folgende Formelschema beispielhaft veranschaulicht werden.

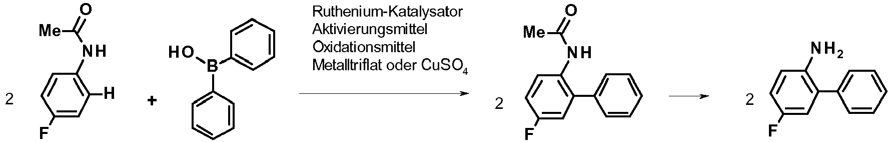

Die Organoborverbindungen der Formel (III) sind prinzipiell bekannt und können nach bekannten Methoden hergestellt werden.

Bevorzugt ist die Durchführung des erfindungsgemäßen Verfahrens unter Verwendung von Ausgangsstoffen, in denen die angegeben Reste jeweils folgende Bedeutungen haben. Die bevorzugten, besonders bevorzugten und ganz besonders bevorzugten Bedeutungen gelten für alle Verbindungen, in denen die jeweiligen Reste vorkommen:
- R¹: steht bevorzugt für Wasserstoff, Fluor und Chlor.
- R¹: steht weiter bevorzugt für Fluor oder Chlor. wobei der Substituent bevorzugt in in 3-, 4- oder 5-Position, weiter bevorzugt in 4- oder 5-Position und besonders bevorzugt in 5- Position steht [vgl. z.B. Formel (I)].
- R¹: steht besonders bevorzugt für Fluor in den oben genannten Positionen, ganz besonders bevorzugt in 4- Position.

In einer alternativen Ausführungsform steht
- R¹: bevorzugt für Trifluormethyl, wobei Trifluormethyl bevorzugt in 4- oder 5-Position, weiter bevorzugt in 5- Position der jeweiligen Verbindung steht.

In einer weiteren alternativen Ausführungsform steht
- R¹: bevorzugt für Methoxy oder Methylthio, bevorzugt in 4-, 5- oder 6-Position, weiter bevorzugt in 5- Position der jeweiligen Verbindung stehen.
- R²: steht bevorzugt für Methyl, Ethyl, iso-Propyl, tert-Butyl, Phenyl oder Benzyl.
- R²: steht weiter bevorzugt für Methyl, Phenyl oder Benzyl.
- R²: steht besonders bevorzugt für Methyl.
- X¹: steht bevorzugt für Wasserstoff, Methyl, Fluor oder Chlor.
- X¹: steht weiter bevorzugt für Fluor oder Chlor.
- X¹: steht besonders bevorzugt für Chlor.
- X²: steht bevorzugt für Wasserstoff ,Methyl, Fluor oder Chlor.
- X²: steht weiter bevorzugt für Fluor oder Chlor.
- X²: steht besonders bevorzugt für Chlor.
- X³: steht bevorzugt für Wasserstoff ,Methyl, Fluor oder Chlor.
- X³: steht weiter bevorzugt für Fluor oder Chlor.
- X³: steht besonders bevorzugt für Chlor.

In einer besonders bevorzugten Ausführungsform steht einer der Substituenten X¹, X² oder X³ für Wasserstoff, wobei besonders bevorzugt benachbarte Substituenten nicht beide gleich Wasserstoff sind.

Bevorzugte Ausführungsformen von Verbindungen der Formel (V) im Sinne der vorliegenden Erfindung sind (die Zahlen zu R¹ geben jeweils die Position an):

| | R¹ | R² | X¹ | X² | X³ |
|---|---|---|---|---|---|
| V1 | H | Me | Cl | Cl | H |
| V2 | H | Me | H | Cl | H |
| V3 | F (4) | Me | Cl | Cl | H |
| V4 | F (3) | Me | Cl | Cl | H |
| V5 | F (5) | Me | Cl | Cl | H |
| V6 | Cl (4) | Me | Cl | Cl | H |
| V7 | Cl (3) | Me | Cl | Cl | H |
| V8 | Cl (5) | Me | Cl | Cl | H |
| V9 | Me (4) | Me | Cl | Cl | H |
| V10 | Me (5) | Me | Cl | Cl | H |
| V11 | F(4) | Me | Cl | Cl | Cl |
| V12 | H | Me | H | H | H |
| V13 | H | Me | F | F | F |
| V14 | OH (5) | Me | H | H | H |
| V15 | Et (5) | Me | H | H | H |
| V16 | OMe (4) | Me | H | H | H |
| V17 | H | Me | H | OMe | H |
| V18 | H | Me | H | Me | H |
| V19 | H | iPr | H | H | H |
| V20 | H | tBu | H | H | H |

Die bei der Durchführung des erfindungsgemäßen Verfahrens in der ersten Stufe als Ausgangsstoffe zu verwendenden Anilide der Formel (II) sind bekannt oder können nach bekannten Verfahren erhalten werden.

Die erste Stufe des erfindungsgemäßen Verfahrens wird in Gegenwart eines Rutheniumkatalysators durchgeführt. Als Rutheniumkatalysator werden beispielsweise Rutheniumkomplexe wie [{RuCl₂(p-Cymol)}₂], [{RuCl₂(Cumol)}₂], [{RuCl₂(Benzol)}₂], [{RuCl₂(C₆Me₆)}₂], [Cp*Ru(PPh₃)₂Cl] (Cp*=Pentamethylcyclopentadienyl) eingesetzt. Bevorzugt verwendet man [{RuCl₂(p-Cymol)}₂].

Die Menge an Rutheniumkatalysator kann in weiten Grenzen variiert werden. Üblicherweise setzt man Mengen von 0,1 bis 20 Molprozent des entsprechenden Komplexes ein. Bevorzugt setzt man 1 bis 10 Molprozent des entsprechenden Komplexes ein.

Bei der Durchführung der ersten Stufe des erfindungsgemäßen Verfahrens wird in Gegenwart eines Aktivierungsmittels gearbeitet, das aus dem eingesetzten Rutheniumkomplex den eigentlich aktiven Katalysator generiert. Als solche Aktivierungsmittel werden üblicherweise AgSbF₆, KPF₆, NaPF₆, AgF, AgBF₄ eingesetzt. Bevorzugt verwendet man AgSbF₆, AgBF₄ und KPF₆, besonders bevorzugt AgSbF₆.

Das Aktivierungsmittel wird in Mengen von 1 bis 4 molaren Äquivalenten, bezogen auf den Rutheniumkomplex, eingesetzt. Bevorzugt werden 1,5 bis 3 Äquivalente eingesetzt.

Bei der Durchführung der ersten Stufe des erfindungsgemäßen Verfahrens wird in Gegenwart mindestens eines Oxidationsmittels gearbeitet, wobei als Oxidationsmittel bevorzugt Ag₂O verwendet wird.

Das Oxidationsmittel wird in Mengen von 0,5 bis 2 molaren Äquivalenten, bezogen auf das Anilid der Formel (II), eingesetzt. Bevorzugt werden 1 bis 2 Äquivalente eingesetzt.

Bei der Durchführung der ersten Stufe des erfindungsgemäßen Verfahrens wird in Gegenwart eines Metalltriflates oder in Gegenwart von Kupfer(II)sulfat oder Kupfer(I)- oder Kupfer(II)oxid gearbeitet. Als Metalltriflate werden Verbindungen wie beispielsweise Kupfer(II)triflat, Mangan(II)triflat, Kobalt(II)triflat), Nickel(II)triflat, Zink(II)triflat, Eisen(II)triflat, Eisen(III)triflat, Lithiumtriflat, Natriumtriflat, Kaliumtriflat, Magnesiumtriflat oder Calciumtriflat eingesetzt. Bevorzugt werden die weiter oben genannten Verbindungen eingesetzt, insbesondere Natriumtriflat, Kaliumtriflat, Mangantriflat, Zinktriflat, Nickel(II)triflat, Eisen(II)triflat und Eisen(III)triflat. Ganz besonders bevorzugt werden Eisentriflate und Nickel(II)triflat verwendet.

Das Metalltriflat (oder Metallsulfat oder Kupfer(I)- oder Kupfer(II)oxid) wird in Mengen von 1 bis 4 molaren Äquivalenten, bezogen auf den Rutheniumkomplex, eingesetzt. Bevorzugt werden 1,5 bis 3 Äquivalente eingesetzt.

Bei der Durchführung der ersten Stufe des erfindungsgemäßen Verfahrens arbeitet man in Lösungsmitteln oder Lösungsmittelgemischen ausgewählt aus der Gruppe, die N,N-Dialkylalkanamide, z.B. N-Methylpyrrolidon (NMP), Dimethylformamid (DMF) und Dimethylacetamid (DMA), Dimethoxyethan (DME), Methanol, Essigsäureethylester und Wasser sowie Mischungen dieser Lösungmittel umfaßt.

Bevorzugte Lösungsmittel oder Lösungsmittelgemische sind solche ausgewählt aus der Gruppe die N,N-Dialkylalkanamide, weiter bevorzugt hieraus N-Methylpyrrolidon (NMP), Dimethylformamid (DMF) und Dimethylacetamid (DMA), und besonders bevorzugt DMF, ganz besonders bevorzugt getrocknetes DMF (Lagerung über Molsieb 4 Angström) umfaßt.

Unter ökologischen Aspekten ist Wasser ein bevorzugtes Lösungsmittel, daß überraschender Weise das Produkt in einer relativ guten Ausbeute lieferte.

Für nicht Triflat-aktivierte Reaktionen alternativer Ausführungsformen kann ebenso THF als Lösungsmittel mit guten Ausbeuten verwendet werden.

Bei der Durchführung der ersten Stufe des erfindungsgemäßen Verfahrens arbeitet man im Allgemeinen bei Temperaturen im Bereich von 20°C bis 200°C, vorzugsweise im Bereich von 50°C bis 150°C.

Bei der Durchführung der ersten Stufe des erfindungsgemäßen Verfahrens setzt man auf 1 Mol an Anilid der Formel (II) im Allgemeinen einen Überschuss an Organoborverbindung der Formel (III) ein.

Die zweite Stufe des erfindungsgemäßen Verfahrens, d.h. die Abspaltung der Schutzgruppe [-C(=O)R²] am Stickstoff; kann entweder basisch oder sauer nach bekannten Methoden erfolgen (vgl. z.B. T.W. Greene, P.G.M. Wuts, Protective Groups in Organic Synthesis, Ed. 3, New York, Wiley & Sons, 1999).

Beide Stufen des erfindungsgemäßen Verfahrens werden, wenn nicht anders angegeben, im Allgemeinen unter Normaldruck durchgeführt. Es ist jedoch auch möglich, unter erhöhtem oder unter vermindertem Druck zu arbeiten.

In einer bevorzugten Ausführungsform des Erfindungsgemäßen Verfahrens ist das Lösemittel ein N,N-Dialkylalkanamid und der das Triflat ist ausgewählt aus der Gruppe die Eisen(III)triflat und Nickel(II)triflat, besonders bevorzugt Eisen(III)triflat umfaßt. Weiterhin ist in dieser Kombination bevorzugt, daß der Katalysator [{RuCl₂(p-Cymol)}₂]. Noch weiter bevorzugt ist der Aktivator AgSbF₆ und das Oxidationsmittel Ag₂O.

Die Biphenylamine der Formel (I) sind wertvolle Zwischenprodukte zur Herstellung fungizider Wirkstoffe (vgl. WO 03/070705).

Das erfindungsgemäße Verfahren soll durch folgende Beispiele veranschaulicht werden, ohne auf diese eingeschränkt zu sein.

### Herstellungsbeispiele

**Beispiel 1:**

### N-([1,1'-Biphenyl]-2-yl)acetamid

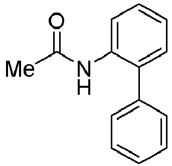

In einem ausgeheizten verschließbaren Reaktionsgefäß wurde eine Suspension bestehend aus
Acetanilid (135 mg, 1.0 mmol), [{RuCl₂(p-Cymol)}₂] (30.6 mg, 5.0 mol %), AgSbF₆(68.7 mg, 0.2 mmol), Ag₂O (232 mg, 1.0 mmol), Cu(OTf)₂ (72.3 mg, 0.2 mmol) und Phenylboronsäure (183 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 162 mg N-([1,1'-Biphenyl]-2-yl)acetamid als farbloser Feststoff erhalten (77% der Theorie). Smp. = 113-115 °C. ¹H-NMR (CDCl₃, 300 MHz): δ = 8.21 (d, J = 8.2 Hz, 1H), 7.51-7.30 (m, 6H), 7.24-7.13 (m, 3H), 1.98 (s, 3H). ¹³C-NMR (CDCl₃, 75 MHz): δ = 168.2 (Cq), 138.1 (Cq), 134.6 (Cq), 132.2 (Cq), 130.0 (CH), 129.1 (CH), 129.0 (CH), 128.2 (CH), 127.9 (CH), 124.3 (CH), 121.7 (CH), 24.4 (CH₃). IR (neat): 3284, 3230, 3054, 3027, 1658, 1531, 1433, 1301, 755, 741, 703, 662, 520 cm⁻¹. MS (EI) m/z (relative Intensität): 211 ([M⁺] 34), 169 (100), 139 (7), 115 (5), 43 (15). HR-MS (ESI) m/z berechnet für C₁₄H₁₃NO [M⁺] 211.0997, gefunden 211.0996.

### Beispiel 2: (Vgl)

### N-([1,1'-Biphenyl]-2-yl)acetamid

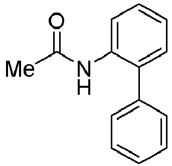

Es wurde vorgegangen wie in Beispiel1 beschrieben, jedoch wurde die Reaktion in THF anstatt in DMF durchgeführt. Es wurden 116 mg N-([1,1'-Biphenyl]-2-yl)acetamid als farbloser Feststoff erhalten (55% der Theorie).

### Beispiel 2a:

### N-([1,1'-Biphenyl]-2-yl)acetamid

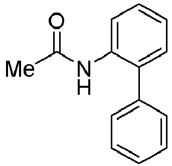

Es wurde vorgegangen wie in Beispiel 1 beschrieben, jedoch wurde die Reaktion in Essigester anstatt in DMF durchgeführt. Es wurden N-([1,1'-Biphenyl]-2-yl)acetamid als farbloser Feststoff erhalten (55% der Theorie).

### Beispiel 2b:

### N-([1,1'-Biphenyl]-2-yl)acetamid

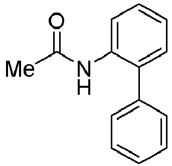

Es wurde vorgegangen wie in Beispiel 1 beschrieben, jedoch wurde die Reaktion in Methanol anstatt in DMF durchgeführt. Es wurden N-([1,1'-Biphenyl]-2-yl)acetamid als farbloser Feststoff erhalten (70% der Theorie).

### Beispiel 2c:

### N-([1,1'-Biphenyl]-2-yl)acetamid

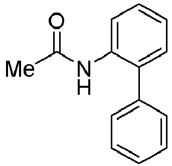

Es wurde vorgegangen wie in Beispiel 1 beschrieben, jedoch wurde die Reaktion in Dichlorethan anstatt in DMF durchgeführt. Es wurden N-([1,1'-Biphenyl]-2-yl)acetamid als farbloser Feststoff erhalten (57% der Theorie).

### Beispiel 2d:

### N-([1,1'-Biphenyl]-2-yl)acetamid

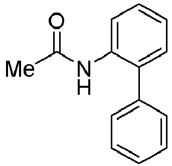

Es wurde vorgegangen wie in Beispiel 1 beschrieben, jedoch wurde die Reaktion in Wasser anstatt in DMF durchgeführt. Es wurden N-([1,1'-Biphenyl]-2-yl)acetamid als farbloser Feststoff erhalten (43% der Theorie).

### Beispiel 2e:

### N-([1,1'-Biphenyl]-2-yl)acetamid

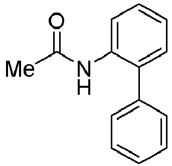

Es wurde vorgegangen wie in Beispiel 1 beschrieben, jedoch wurde die Reaktion in DMA anstatt in DMF durchgeführt. Es wurden N-([1,1'-Biphenyl]-2-yl)acetamid als farbloser Feststoff erhalten (46% der Theorie).

### Beispiel 2f:

### N-([1,1'-Biphenyl]-2-yl)acetamid

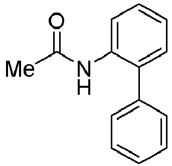

Es wurde vorgegangen wie in Beispiel 1 beschrieben, jedoch wurde die Reaktion in DME anstatt in DMF durchgeführt. Es wurden N-([1,1'-Biphenyl]-2-yl)acetamid als farbloser Feststoff erhalten (50% der Theorie).

### Beispiel 2g:

### N-([1,1'-Biphenyl]-2-yl)acetamid

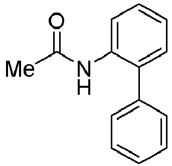

Es wurde vorgegangen wie in Beispiel 1 beschrieben, jedoch wurde die Reaktion in einem 1:1-Gemisch aus DMF und THF anstatt in DMF durchgeführt. Es wurden N-([1,1'-Biphenyl]-2-yl)acetamid als farbloser Feststoff erhalten (65% der Theorie).

### Beispiel 3a:

### N-([1,1'-Biphenyl]-2-yl)acetamid

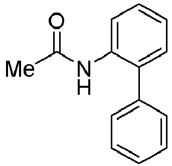

In einem ausgeheizten verschließbaren Reaktionsgefäß wurde eine Suspension bestehend aus
Acetanilid (135 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Fe(OTf)₃ (101 mg, 0.2 mmol) und Phenylboronsäure (183 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 171 mg N-([1,1'-Biphenyl]-2-yl)acetamid als farbloser Feststoff erhalten (81% der Theorie).

### Beispiel 3b:

### N-([1,1'-Biphenyl]-2-yl)acetamid

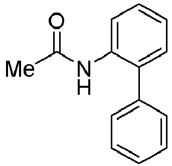

Es wurde vorgegangen wie in Beispiel 3a beschrieben, jedoch wurde die Reaktion in THF anstatt in DMF durchgeführt. Es wurde N-([1,1'-Biphenyl]-2-yl)acetamid in einer Ausbeute von 56% der Theorie erhalten.

### Beispiel 4:

### N-([1,1'-Biphenyl]-2-yl)acetamid

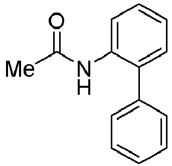

In einem ausgeheizten verschließbaren Reaktionsgefäß wurde eine Suspension bestehend aus Acetanilid (135 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und Diphenylborinsäure (137 mg, 0.75 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 84.4 mg N-([1,1'-Biphenyl]-2-yl)acetamid als farbloser Feststoff erhalten (40% der Theorie).

### Beispiel 5:

### N-(4-Methyl-[1,1'-biphenyl]-2-yl)acetamid

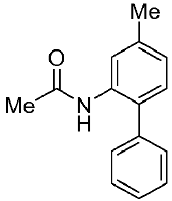

In einem ausgeheizten verschließbaren Reaktionsgefäß wurde eine Suspension bestehend aus N-(m-Tolyl)acetamid (149 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und Phenylboronsäure (183 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 175 mg N-(4-Methyl-[1,1'-biphenyl]-2-yl)acetamid als farbloser Feststoff erhalten (78% der Theorie). Smp. = 139-141 °C. ¹H-NMR (CDCl₃, 300 MHz): δ = 8.04 (s, 1H), 7.50-7.28 (m, 5H), 7.21-7.08 (m, 2H), 6.98 (d, J = 7.6 Hz, 1H), 2.38 (s, 3H), 1.98 (s, 3H). ¹³C-NMR (CDCl₃, 125 MHz): δ = 168.0 (Cq), 138.3 (Cq), 138.1 (Cq), 134.3 (Cq), 129.7 (CH), 129.4 (Cq), 129.2 (CH), 128.9 (CH), 127.6 (CH), 125.1 (CH), 122.2 (CH), 24.6 (CH₃), 21.5 (CH₃). IR (neat): 3224, 3029, 2916, 1652, 1539, 1476, 1412, 1297, 820, 763, 724, 700, 611, 524 cm⁻¹. MS (EI) m/z (relative Intensität): 225 ([M⁺] 54), 183 (100), 167 (30), 43 (20). HR-MS (ESI) m/z berechnet für C₁₅H₁₅NO [M⁺] 225.1154, gefunden 225.1159.

### Beispiel 6:

### N-(5-Methyl-[1,1'-biphenyl]-2-yl)acetamid

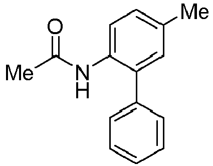

In einem ausgeheizten verschließbaren Reaktionsgefäß wurde eine Suspension bestehend aus
N-(p-Tolyl)acetamid (149 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und Phenylboronsäure (183 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 178 mg N-(5-Methyl-[1,1'-biphenyl]-2-yl)acetamid als farbloser Feststoff erhalten (79% der Theorie). Smp. = 107-109 °C. ¹H-NMR (CDCl₃, 300 MHz): δ = 8.06 (d, J = 8.3 Hz, 1H), 7.50-7.31 (m, 5H), 7.16 (dd, J = 8.3, 2.2 Hz, 1H), 7.04 (m, 2H), 2.33 (s, 3H), 1.99 (s, 3H). ¹³C-NMR (CDCl₃, 126 MHz): δ = 168.0 (Cq), 138.3 (Cq), 134.0 (Cq), 132.3 (Cq), 132.0 (Cq), 131.0 (CH), 129.1 (CH), 128.9 (CH), 128.8 (CH), 127.7 (CH), 121.9 (CH), 24.5 (CH₃), 20.9 (CH₃). IR (neat): 3235, 3057, 3029, 2922, 1655, 1524, 1505, 1488, 1366, 761, 734, 691, 603, 580 cm⁻¹. MS (EI) m/z (relative Intensität): 225 ([M⁺] 54), 183 (100), 167 (18), 43 (22). HR-MS (ESI) m/z berechnet für C₁₅H₁₅NO [M+] 225.1154, gefunden 225.1154.

### Beispiel 7:

### N-(3',4'-Dichlor-5-fluor-[1,1'-biphenyl]-2-yl)acetamid

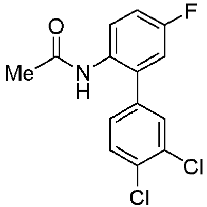

In einem ausgeheizten verschließbaren Reaktionsgefäß wurde eine Suspension bestehend aus N-(4-Fluorphenyl)acetamid (153 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und 3,4-Dichlorphenylboronsäure (286 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 188 mg N-(3',4'-Dichlor-5-fluor-[1,1'-biphenyl]-2-yl)acetamid als farbloser Feststoff erhalten (63% der Theorie). Smp. = 146-148 °C. ¹H-NMR (CDCl₃, 300 MHz): δ = 8.02-7.94 (m, 1H), 7.53 (d, J = 8.2 Hz, 1H), 7.44 (d, J = 2.0 Hz, 1H), 7.18 (dd, J = 8.2, 2.1 Hz, 1H), 7.12-7.01 (m, 1H), 6.96-6.93 (m, 2H), 2.02 (s, 3H). ¹³C-NMR (CDCl₃, 75 MHz): δ = 168.5 (Cq), 159.5 (Cq, J_{C-F} = 246.4 Hz), 137.2 (Cq, J_{C-F} = 1.6 Hz), 133.3 (Cq), 133.0 (Cq, J_{C-F} = 7.6 Hz), 132.8 (Cq), 131.0 (CH), 130.9 (CH), 130.4 (Cq, J_{C-F} = 2.7 Hz), 128.2 (CH), 125.4 (CH, J_{C-F} = 8.0 Hz), 116.5 (CH, J_{C-F} = 23.2 Hz), 115.7 (CH, J_{C-F} = 21.9 Hz), 24.2 (CH₃). ¹⁹F-NMR (282 MHz, CDCl₃) δ = -116.6 (s). IR (neat): 3242, 3190, 1652, 1529, 1472, 1371, 1183, 863, 823, 702, 685, 607, 501 cm⁻¹. MS (EI) m/z (relative Intensität): 297 ([M⁺] 48), 255 (100), 219 (40), 185 (52), 157 (17), 43 (60). HR-MS (ESI) m/z berechnet für C₁₄H₁₀Cl₂FNO [M⁺] 297.0123, gefunden 297.0128.

### Beispiel 8:

### N-([1,1'-Biphenyl]-2-yl)acetamid

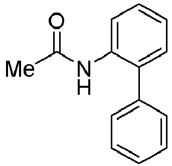

In einem ausgeheizten verschließbaren Reaktionsgefäß wurde eine Suspension bestehend aus
Acetanilid (135 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Na(OTf) (34,4 mg, 0.2 mmol) und Phenylboronsäure (183 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt enthielt lt. NMR-Analyse 65% der Theorie an N-([1,1'-Biphenyl]-2-yl)acetamid.

### Beispiel 9:

### N-([1,1'-Biphenyl]-2-yl)acetamid

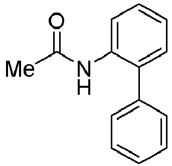

Es wurde vorgegangen wie in Beispiel 8 beschrieben, jedoch wurde die Reaktion in Gegenwart von 0,2 mmol Zn(OTf)₂ anstatt NaOTf durchgeführt. Es wurde N-([1,1'-Biphenyl]-2-yl)acetamid in einer Ausbeute von 74% der Theorie erhalten.

### Beispiel 10:

### N-([1,1'-Biphenyl]-2-yl)acetamid

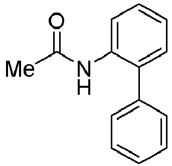

Es wurde vorgegangen wie in Beispiel 8 beschrieben, jedoch wurde die Reaktion in Gegenwart von 0,2 mmol Mn(OTf)₂ anstatt NaOTf durchgeführt. Es wurde N-([1,1'-Biphenyl]-2-yl)acetamid in einer Ausbeute von 76% der Theorie erhalten.

### Beispiel 11:

### N-([1,1'-Biphenyl]-2-yl)acetamid

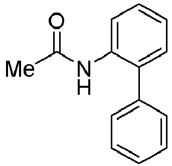

Es wurde vorgegangen wie in Beispiel 8 beschrieben, jedoch wurde die Reaktion in Gegenwart von 0,2 mmol Ni(OTf)₂ anstatt NaOTf durchgeführt. Es wurde N-([1,1'-Biphenyl]-2-yl)acetamid in einer Ausbeute von 82% der Theorie erhalten.

### Beispiel 12:

### N-([1,1'-Biphenyl]-2-yl)acetamid

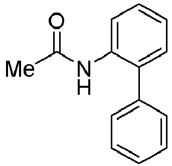

In einem ausgeheizten verschließbaren Reaktionsgefäß wurde eine Suspension bestehend aus
Acetanilid (135 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), CuSO₄ (31,9 mg, 0.2 mmol) und Phenylboronsäure (183 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 91 mg N-([1,1'-Biphenyl]-2-yl)acetamid als farbloser Feststoff erhalten (43% der Theorie

### Beispiel 13:

### N-(3',4',5'-Trifluor-[1,1'-biphenyl]-2-yl)acetamid

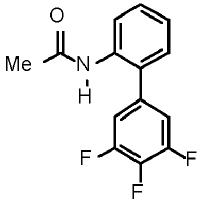

In einem ausgeheizten verschließbaren Reaktionsgefäß wurde eine Suspension bestehend aus
Acetanilid (135 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und 3,4,5-Trifluorhenylboronsäure (264 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 180 mg N-(3',4',5'-Trifluor-[1,1'-biphenyl]-2-yl)acetamid als farbloser Feststoff erhalten (68% der Theorie). M.p. = 140-141 °C. ¹H-NMR (CDCl₃, 300 MHz): δ = 8.08 (d, *J =* 8.1 Hz, 1H), 7.40 (ddd, *J =* 8.5, 5.9, 3.1 Hz, 1H), 7.24-7.17 (m, 2H), 7.06-6.97 (m, 2H), 6.93 (s, 1H), 2.07 (s, 3H). ¹³C-NMR (CDCl₃, 126 MHz): δ = 168.1 (C_{q}) ,151.39 (ddd, *J*_{C-F} = 251.6, 10.0, 4.2 Hz) (Cq), 139.5 (dt, *J*_{C-F} = 253.1, 15.0 Hz) (Cq), 134.5 (Cq), 134.5 (Cq), 130.5 (Cq), 129.8 (CH), 129.5 (CH), 125.1 (CH), 123.3 (CH), 113.5 (dd, *J*_{C-F} = 16.1, 5.4 Hz) (CH), 24.3 (CH₃). ¹⁹F-NMR (282 MHz, CDCl₃) δ = -132-8-133.0 (m), -161.0 (tt, *J*_{C-F} = 20.6, 6.5 Hz). IR (neat): 3263, 3040, 2934, 2864, 1660, 1526, 1483, 1417, 1359, 1278, 1241, 1036, 872, 857, 762, 695, 669, 634, 606, 547, 465 cm⁻¹. MS (EI) *m*/*z* (relative Intensität): 265 ([M⁺] 29), 223 (100), 203 (16), 175 (5), 169 (5), 84 (6), 43 (41). HR-MS (ESI) *m*/*z* berechnet für C₁₄H₁₀F₃NO [M⁺] 265.0714, gefunden 265.0718.

### Beispiel 14:

### N-(4'-Chlor-[1,1'-biphenyl]-2-yl)acetamid

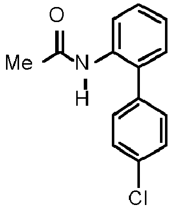

In einem ausgeheizten verschließbaren Reaktionsgefäß wurde eine Suspension bestehend aus
Acetanilid (135 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und 4-Chlorphenylboronsäure (234 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 172 mg N-(4'-Chlor-[1,1'-biphenyl]-2-yl)acetamid als farbloser Feststoff erhalten (70% der Theorie).
M.p. = 114-116 °C. ¹H-NMR (CDCl₃, 300 MHz): δ=8.16 (d, *J* = 8.2 Hz, 1H), 7.46-7.40 (m, 2H), 7.39-7.32 (m, 1H), 7.31-7.26 (m, 2H), 7.20-7.17 (m, 2H), 7.01 (s, 1H), 2.01 (s, 3H). ¹³C-NMR (CDCl₃, 126 MHz): δ = 168.1 (Cq), 136.5 (Cq), 134.4 (Cq), 134.0 (Cq), 131.3 (Cq), 130.5 (CH), 129.9 (CH), 129.1 (CH), 128.6 (CH), 124.6 (CH), 122.2 (CH), 24.6 (CH₃). IR (neat): 3247, 3031, 2924, 2854, 1635, 1527, 1369, 1283, 1086, 828, 756, 607, 530, 489 cm⁻¹. MS (EI) *m*/*z* (relative Intensität): 245 ([M⁺] 35), 203 (100), 167 (43), 139 (12), 84 (17), 43 (36). HR-MS (ESI) *m*/*z* berechnet für C₁₄H₁₂ClNO [M⁺] 245.0607, gefunden 245.0599.

### Beispiel 15:

### N-(5-Methoxy-[1,1'-biphenyl]-2-yl)acetamid

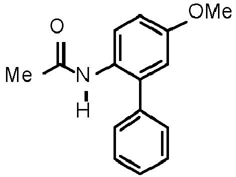

In einem ausgeheizten verschließbaren Reaktionsgefäß wurde eine Suspension bestehend aus
4-Methoxy-acetanilid (165 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und 4-Chlorphenylboronsäure (234 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 183 mg N-(5-Methoxy-[1,1'-biphenyl]-2-yl)acetamid als farbloser Feststoff erhalten (76% der Theorie). M.p. = 112-114 °C. ¹H-NMR (CDCl₃, 300 MHz): δ = 7.95 (d, *J* = 8.9 Hz, 1H), 7.50-7.28 (m, 5H), 6.98 (s, 1H), 6.88 (dd, *J* = 8.9, 3.0 Hz, 1H), 6.78 (d, *J* = 3.0 Hz, 1H), 3.78 (s, 3H), 1.97 (s, 3H). ¹³C-NMR (CDCl₃, 126 MHz): δ = 168.2 (Cq), 156.3 (Cq), 138.1 (Cq), 134.7 (Cq), 128.9 (CH), 128.8 (CH), 127.8 (CH), 127.6 (Cq), 124.3 (CH), 115.3 (CH), 113.3 (CH), 55.5 (CH₃), 24.2 (CH₃).

IR (neat): 3263, 3058, 2969, 2939, 2838, 1664, 1480, 1270, 1207, 1178, 1033, 701, 599, 512 cm⁻¹. MS (EI) *m*/*z* (relative Intensität): 241 ([M⁺] 71), 199 (76), 184 (100), 154 (21), 128 (11), 43 (34). HR-MS (ESI) *m*/*z* berechnet für C₁₅H₁₅NO₂ [M⁺] 241.1103, gefunden 241.1106.

### Beispiel 16:

### N-(4-Methoxy-[1,1'-biphenyl]-2-yl)acetamid

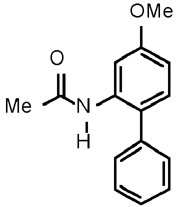

In einem ausgeheizten verschließbaren Reaktionsgefäß wurde eine Suspension bestehend aus
3-Methoxy-acetanilid (165 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und 4-Chlorphenylboronsäure (234 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 174 mg N-(4-Methoxy-[1,1'-biphenyl]-2-yl)acetamid als farbloser Feststoff erhalten (72% der Theorie). M.p. = 91-93 °C. ¹H-NMR (CDCl₃, 300 MHz): δ = 7.98 (d, *J* = 2.6 Hz, 1H), 7.49-7.42 (m, 2H), 7.40-7.36 (m, 1H), 7.35-7.32 (m, 1H), 7.32-7.29 (m, 1H), 7.17 (s, 1H), 7.12 (d, *J* = 8.5 Hz, 1H), 6.72 (dd, *J* = 8.5, 2.6 Hz, 1H), 3.84 (s, 3H), 2.00 (s, 3H). ¹³C-NMR (CDCl₃, 126 MHz): δ = 168.1 (Cq), 159.4 (Cq), 137.9 (Cq), 135.6 (Cq), 130.6 (CH), 129.3 (CH), 129.0 (CH), 127.6 (CH), 124.2 (Cq), 110.5 (CH), 106.2 (CH), 55.5 (CH₃), 24.8 (CH₃). IR (neat): 3415, 3241, 3033, 2953, 2831, 1652, 1309, 1233, 762, 724, 698, 621, 525 cm⁻¹. MS (EI) *m*/*z* (relative Intensität): 241 ([M⁺] 74), 199 (100), 170 (16), 156 (19), 84 (9), 43 (34). HR-MS (ESI) *m*/*z* berechnet für C₁₅H₁₅NO₂ [M⁺] 241.1103, found 241.1107.

### Beispiel 17:

### N-(5-Ethyl-[1,1'-biphenyl]-2-yl)acetamid

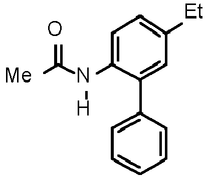

In einem ausgeheizten verschließbaren Reaktionsgefäß wurde eine Suspension bestehend aus
4-Ethyl-acetanilid (163 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und Phenylboronsäure (183 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 151 mg N-(5-Ethyl-[1,1'-biphenyl]-2-yl)acetamid als farbloser Feststoff erhalten (63% der Theorie). M.p. = 64-65 °C. ¹H-NMR (CDCl₃, 300 MHz): δ = 8.07 (d, *J =* 8.3 Hz, 1H), 7.54-7.31 (m, 5H), 7.20 (dd, *J =* 8.3, 2.3 Hz, 1H), 7.13 (s, 1H), 7.09 (d, *J* = 2.3 Hz, 1H), 2.65 (q, *J* = 7.6 Hz, 2H), 2.00 (s, 3H), 1.25 (t, *J* = 7.6 Hz, 3H). ¹³C-NMR (CDCl₃, 126 MHz): δ = 168.0 (Cq), 140.3 (Cq), 138.3 (Cq), 132.5 (Cq), 132.1 (Cq), 129.3 (CH), 129.0 (CH), 128.8 (CH), 127.6 (CH), 127.5 (CH), 122.2 (CH), 28.3 (CH₂), 24.4 (CH₃), 15.6 (CH₃). IR (neat): 3424, 3267, 3027, 2964, 2930, 2871, 1659, 1513, 1487, 1410, 1368, 1297, 767, 699, 509 cm⁻¹. MS (EI) *m*/*z* (relative Intensität): 239 ([M⁺] 58), 197 (58), 182 (100), 180 (19), 167 (16), 43 (37). HR-MS (ESI) *m*/*z* berechnet für C₁₆H₁₇NO [M⁺] 239.1310, gefunden 239.1306.

### Beispiel 18:

### N-(5-Hydroxy-[1,1'-biphenyl]-2-yl)acetamid

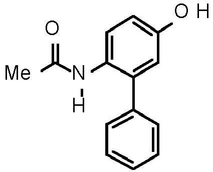

In einem ausgeheizten verschließbaren Reaktionsgefäß wurde eine Suspension bestehend aus
4-Hydroxy-acetanilid (151 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und Phenylboronsäure (183 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 157 mg N-(5-Hydroxy-[1,1'-biphenyl]-2-yl)acetamid als farbloser Feststoff erhalten (69% der Theorie). ¹H-NMR (CDCl₃, 300 MHz): δ = 7.59 (d, *J* = 9.5 Hz, 1H), 7.53 (s, 1H), 7.43-7.32 (m, 3H), 7.30-7.24 (m, 2H), 7.05 (s, 1H), 6.73-6.67 (m, 2H), 1.99 (s, 3H). ¹³C-NMR (CDCl₃, 126 MHz): δ = 169.7 (Cq), 154.1 (Cq), 138.1 (Cq), 136.1 (Cq), 128.8 (CH), 128.6 (CH), 127.6 (CH), 126.0 (Cq), 125.7 (CH), 117.1 (CH), 115.3 (CH), 23.9 (CH₃). IR (neat): 3268, 3057, 2959, 2926, 2795, 1524, 1488, 1433, 1299, 1199, 726, 699, 646, 506 cm⁻¹. MS (EI) *m*/*z* (relative Intensität): 227 ([M⁺] 44), 185 (100), 154 (11), 43 (14). HR-MS (ESI) *m*/*z* berechnet für C₁₄H₁₃NO₂ [M⁺] 227.0946, gefunden 227.0945.

### Beispiel 19:

### N-(4'-Methyl-[1,1'-biphenyl]-2-yl)acetamid

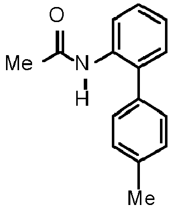

In einem ausgeheizten verschließbaren Reaktionsgefäß wurde eine Suspension bestehend aus
Acetanilid (135 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und 4-Methylphenylboronsäure (204 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 185 mg N-(4'-Methyl-[1,1'-biphenyl]-2-yl)acetamid als farbloser Feststoff erhalten (82% der Theorie). M.p. = 106-108 °C. ¹H-NMR (CDCl₃, 300 MHz): δ = 8.24 (d, *J* = 8.2 Hz, 1H), 7.44-7.06 (m, 8H), 2.41 (s, 3H), 2.01 (s, 3H). ¹³C-NMR (CDCl₃, 126 MHz): δ = 168.0 (Cq), 137.6 (Cq), 135.0 (Cq), 134.6 (Cq), 132.0 (Cq), 130.0 (CH), 129.7 (CH), 128.9 (CH), 128.1 (CH), 124.1 (CH), 121.4 (CH), 24.6 (CH₃), 21.2 (CH₃). IR (neat): 3340, 2956, 2921, 2853, 1515, 1442, 1282, 817, 756, 680, 598, 522, 488 cm⁻¹. MS (EI) *m*/*z* (relative Intensität): 225 ([M⁺] 55), 183 (100), 167 (37), 43 (26). HR-MS (ESI) *m*/*z* berechnet für C₁₅H₁₅NO [M⁺] 225.1154, gefunden 225.1149.

### Beispiel 20

### N-(4'-Methoxy-[1,1'-biphenyl]-2-yl)acetamid

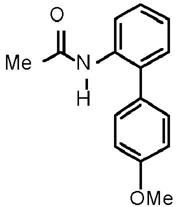

In einem ausgeheizten verschließbaren Reaktionsgefäß wurde eine Suspension bestehend aus
Acetanilid (135 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und 4-Methoxyphenylboronsäure (228 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 200 mg N-(4'-Methyl-[1,1'-biphenyl]-2-yl)acetamid als farbloser Feststoff erhalten (83% der Theorie). M.p. = 135-137 °C. ¹H-NMR (CDCl₃, 300 MHz): δ = 8.20 (d, *J* = 8.2 Hz, 1H), 7.34-7.24 (m, 3H), 7.23-7.09 (m, 3H), 6.98 (d, *J* = 8.6 Hz, 2H), 3.84 (s, 3H), 2.00 (s, 3H). ¹³C-NMR (CDCl₃, 126 MHz): δ = 168.3 (Cq), 159.3 (Cq), 134.8 (Cq), 132.0 (Cq), 130.3 (CH), 130.2 (Cq), 130.1 (CH), 128.0 (CH), 124.3 (CH), 121.6 (CH), 114.4 (CH), 55.2 (CH₃), 24.4 (CH₃). IR (neat): 3351, 3012, 2921, 2842, 1690, 1602, 1512, 1439, 1362, 1294, 1239, 1175, 1031, 832, 800, 770, 663, 581, 560, 534 cm⁻¹. MS (EI) *m*/*z* (relative Intensität): 241 ([M⁺] 54), 199 (100), 184 (37), 154 (24), 128 (12), 43 (30). HR-MS (ESI) *m*/*z* berechnet für C₁₅H₁₅NO₂ [M⁺] 241.1103, gefunden 241.1110.

### Beispiel 21

### N-(Biphenyl-2-yl)-2-methylpropanamid

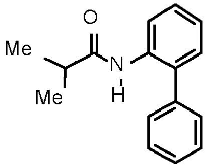

In einem ausgeheizten verschließbaren Reaktionsgefäß wurde eine Suspension bestehend aus
2-Methyl-N-phenylpropanamid (163 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und Phenylboronsäure (183 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 131 mg N-(Biphenyl-2-yl)-2-methylpropanamid als farbloser Feststoff erhalten (55% der Theorie). M.p. = 126-128 °C. ¹H-NMR (CDCl₃, 300 MHz): δ = 8.33 (d, *J* = 8.2 Hz, 1H), 7.54-7.33 (m, 6H), 7.28-7.13 (m, 3H), 2.4 (hept, *J* = 6.8 Hz, 1H), 1.2 (d, *J* = 6.8 Hz, 6H). ¹³C-NMR (CDCl₃, 126 MHz): δ = 174.8 (Cq), 138.1 (Cq), 134.9 (Cq), 132.1 (Cq), 129.9 (CH), 129.3 (CH), 129.0 (CH), 128.4 (CH), 128.0 (CH), 124.0 (CH), 121.3 (CH), 36.7 (CH), 19.3 (CH₃). IR (neat): 3218, 2964, 1649, 1520, 1480, 1239, 1203, 1099, 776, 748, 726, 702, 542 cm⁻¹. MS (EI) *m*/*z* (relative Intensität): 239 ([M⁺] 29), 169 (100), 71 (6), 43 (30). HR-MS (ESI) *m*/*z* berechnet für C₁₆H₁₇NO [M⁺] 239.1310, gefunden 239.1314.

### Beispiel 22

### N-(Biphenyl-2-yl)-2,2-dimethylpropanamid

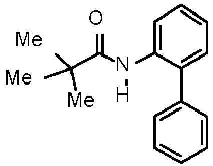

In einem ausgeheizten verschließbaren Reaktionsgefäß wurde eine Suspension bestehend aus
2,2-Dimethyl-N-phenylpropanamid (177 mg, 1.0 mmol), [{RuCl₂(p-Cymol)}₂] (30.6 mg, 5.0 mol %), AgSbF₆ (68.7 mg, 0.2 mmol), Ag₂O (232 mg, 1.0 mmol), Cu(OTf)₂ (72.3 mg, 0.2 mmol) und Phenylboronsäure (183 mg, 1.5 mmol) in trockenem DMF (3.0 mL) bei 110 °C für 20 h in einer Stickstoffatmosphäre gerührt. Das Reaktionsgemisch wurde dann bei Raumtemperatur mit EtOAc (75 mL) verdünnt, über Celite und Kieselgel filtriert und das Filtrat eingeengt. Das so erhaltene Rohprodukt wurde durch Chromatografie an Kieselgel gereinigt (n-Hexan/EtOAc: 7/3). Es wurden 114 mg N-(Biphenyl-2-yl)-2,2-dimethylpropanamid als farbloser Feststoff erhalten (45% der Theorie). M.p. = 68-69 °C. ¹H-NMR (CDCl₃, 300 MHz): δ = 8.37 (dd, J= 8.2, 1.2 Hz, 1H), 7.54-7.33 (m, 7H), 7.24 (dd, J= 7.4, 1.7 Hz, 1H), 7.17 (dd, *J* = 7.4, 1.7 Hz, 1H), 1.09 (s, 9H). ¹³C-NMR (CDCl₃, 126 MHz): δ = 176.1 (Cq), 138.0 (Cq), 135.0 (Cq), 132.0 (Cq), 129.6 (CH), 129.2 (CH), 128.9 (CH), 128.4 (CH), 127.9 (CH), 123.8 (CH), 120.8 (CH), 39.8 (Cq), 27.4 (CH₃). IR (neat): 3259, 3056, 2970, 2904, 2868, 1646, 1503, 1477, 771, 743, 700, 647 cm⁻¹. MS (EI) *m*/*z* (relative Intensität): 253 ([M⁺] 53), 169 (60), 57 (100), 41 (17). HR-MS (ESI) *m*/*z* berechnet für C₁₇H₁₉NO [M⁺] 253.1467, gefunden 253.1472.

## Patentansprüche

1. Verfahren zur Herstellung von Biphenylamiden der allgemeinen Formel (V) 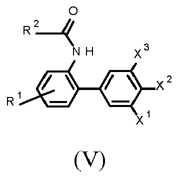 in welcher
R¹ für Wasserstoff, Hydroxy, Fluor, Chlor, C₁-C₄-Alkyl, C₁-C₄-Alkoxy, C₁-C₄-Alkylthio oder C₁-C₄-Halogenalkyl steht,
R² für für C₁-C₄-Alkyl, C₆-C₁₀-Aryl oder C₆-C₁₀-Aryl-CH₂- steht, und
X¹ für Wasserstoff, C₁-C₄-Alkyl, C₁-C₄-Alkoxy, Fluor oder Chlor steht,
X² für Wasserstoff, C₁-C₄-Alkyl, C₁-C₄-Alkoxy, Fluor oder Chlor steht,
X³ für Wasserstoff, C₁-C₄-Alkyl, C₁-C₄-Alkoxy, Fluor oder Chlor steht,
**dadurch gekennzeichnet, dass** man
Anilide der Formel (II) 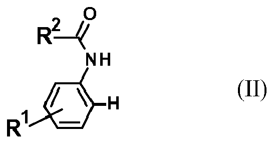 in welcher
R¹ und R² die oben angegebenen Bedeutungen haben,
in einem von Tetrahydrofuran verschiedenen Lösungsmittel
mit einer Organoborverbindung der Formel (III) 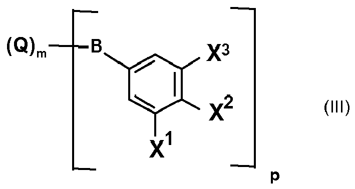 in welcher
X¹, X² und X³ die oben angegebenen Bedeutungen haben,
und die aus einer der folgenden Gruppen gewählt ist, bestehend aus
(I) Boronsäuren der Formel (III), in der
Q für eine Hydroxygruppe steht,
m für die Zahl 2 steht,
p für die Zahl 1 steht,
oder den Anhydriden, Dimeren oder Trimeren dieser Boronsäuren;
(II) Boronsäurederivaten der Formel (III), in der
Q für F, Cl, Br, I, C₁-C₄-Alkyl, C₆-C₁₀-Aryl, C₁-C₄-Alkoxy oder C₆-C₁₀-Aryloxy steht,
m für die Zahl 2 steht,
p für die Zahl 1 steht;
(III) Borinsäuren der Formel (III), in der
Q für OH, F, Cl, Br, I, C₁-C₄-Alkyl, C₆-C₁₀-Aryl, C₁-C₄-Alkoxy oder C₆-C₁₀-Aryloxy steht,
m für die Zahl 1 steht,
p für die Zahl 2 steht;
(IV) cyclische Boronsäureester der Formel (III), in der
Q für einen C₂-C₃-Alkyldioxy-Rest steht, der zusammen mit dem Boratom, an das er gebunden ist, einen 5- oder 6-gliedrigen Ring bildet, der gegebenfalls durch einen oder mehrere C₁-C₄-AlkylReste substituiert ist,
m für die Zahl 2 steht,
p für die Zahl 1 steht;
(V) Boronate der Formel (III), in der
Q für OH, F, Cl, Br, I, C₁-C₄-Alkyl, C₆-C₁₀-Aryl, C₁-C₄-Alkoxy oder C₆-C₁₀-Aryloxy steht,
m für die Zahl 3 steht,
p für die Zahl 1 steht
und die negative Ladung des Boronatanions durch ein Kation ausgeglichen wird,
(VI) Triarylborane der Formel (III), in der
m für die Zahl 0 steht,
p für die Zahl 3 steht;
(VII) Tetraarylborate der Formel (IV), in der
m für die Zahl 0 steht,
p für die Zahl 4 steht
und die negative Ladung des Tetraarylboratanions durch ein Kation ausgeglichen wird,
in Gegenwart eines Katalysatorsystems bestehend aus einem Rutheniumkatalysator, einem Aktivierungsmittel, einem Oxidationsmittel und einem Metalltriflat umsetzt.

2. Verfahren nach Anspruch 1, **dadurch gekennzeichnet, daß** in einer zweiten Stufe das Amid der Formel (Ia) zum freien Amin der allgemeinen Formel (I) entschützt wird.

3. Verfahren nach Anspruch 1 oder 2, **dadurch gekennzeichnet, daß** das Lösungsmittel ausgewählt ist aus der Gruppe, die N,N-Dialkylalkanamide, Dimethoxyethan (DME), Methanol, Essigsäureethylester und Wasser sowie Mischungen dieser Lösungmittel umfaßt.

4. Verfahren nach einem der vorhergehenden Ansprüche, **dadurch gekennzeichnet, dass** das Lösungsmittel ausgewählt ist aus der Gruppe, die N,N-Dialkylalkanamide sowie Mischungen dieser Lösungmittel umfasst.

5. Verfahren nach einem der vorhergehenden Ansprüche, **dadurch gekennzeichnet, dass** der Katalysator [{RuCl₂(p-Cymol)}₂] ist.

6. Verfahren nach einem der vorhergehenden Ansprüche, **dadurch gekennzeichnet, dass** das Oxidationsmittel Ag₂O ist.

7. Verfahren nach einem der vorhergehenden Ansprüche, **dadurch gekennzeichnet, daß** das Metalltriflat ausgewählt ist aus der Gruppe, die Li, Na, K, Mg, Ca, Mn, Fe, Co, Ni, Cu und Zn umfaßt.

8. Verfahren nach einem der vorhergehenden Ansprüche, **dadurch gekennzeichnet, daß** das Metalltriflat ausgewählt ist aus der Gruppe, die Natrium, Kalium, Mangan, Zink und Eisen und Nickel umfasst.

9. Verfahren nach einem der vorhergehenden Ansprüche, **dadurch gekennzeichnet, daß** das Aktivierungsmittel ausgewählt ist aus der Gruppe, die AgSbF₆, KPF₆, NaPF₆, AgF und AgBF₄ umfasst.

10. Verfahren nach einem der vorhergehenden Ansprüche, **dadurch gekennzeichnet, dass** die Boronsäure eine Boronsäure der Formel (III) mit Q=OH, M=2 und P=1 ist.

11. Verfahren nach einem der vorhergehenden Ansprüche, **dadurch gekennzeichnet, daß** die Boronsäure eine Boronsäure der Formel (III) mit Q=OH, M=2 und P=1, das Lösungsmittel DMF, der Katalysator [{RuCl₂(p-Cymol)}₂], das Metalltriflat Eisen(III)triflat, das Oxidationsmittel Ag₂O und der Aktivator AgSbF₆ ist.

12. Verfahren zur Herstellung von Biphenylamiden der allgemeinen Formel (Ia) 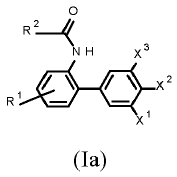 in welcher
R¹ für Wasserstoff, Hydroxy, Fluor, Chlor, C₁-C₄-Alkyl, C₁-C₄-Alkoxy, C₁-C₄-Alkylthio oder C₁-C₄-Halogenalkyl steht,
R² für für C₁-C₄-Alkyl, C₆-C₁₀-Aryl oder C₆-C₁₀-Aryl-CH₂- steht, und
X¹ für Wasserstoff, C₁-C₃-Alkyl, C₁-C₄-Alkoxy, Fluor oder Chlor steht,
X² für Wasserstoff, C₁-C₃-Alkyl, C₁-C₄-Alkoxy, Fluor oder Chlor steht,
X³ für Wasserstoff, C₁-C₃-Alkyl, C₁-C₄-Alkoxy, Fluor oder Chlor steht,
**dadurch gekennzeichnet, dass** man
Anilide der Formel (II) 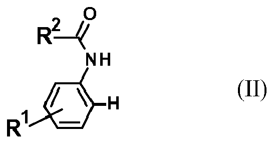 in welcher
R¹ und R² die oben angegebenen Bedeutungen haben,
mit einer Organoborverbindung der Formel (III) 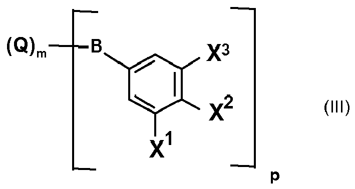 in welcher
X¹, X² und X³ die oben angegebenen Bedeutungen haben,
und die aus einer der folgenden Gruppen gewählt ist, bestehend aus
(I) Boronsäuren der Formel (III), in der
Q für eine Hydroxygruppe steht,
m für die Zahl 2 steht,
p für die Zahl 1 steht,
oder den Anhydriden, Dimeren oder Trimeren dieser Boronsäuren;
(II) Boronsäurederivaten der Formel (III), in der
Q für F, Cl, Br, I, C₁-C₄-Alkyl, C₆-C₁₀-Aryl, C₁-C₄-Alkoxy oder C₆-C₁₀-Aryloxy steht,
m für die Zahl 2 steht,
p für die Zahl 1 steht;
(III) Borinsäuren der Formel (III), in der
Q für OH, F, Cl, Br, I, C₁-C₄-Alkyl, C₆-C₁₀-Aryl, C₁-C₄-Alkoxy oder C₆-C₁₀-Aryloxy steht,
m für die Zahl 1 steht,
p für die Zahl 2 steht;
(IV) cyclische Boronsäureester der Formel (III), in der
Q für einen C₂-C₃-Alkyldioxy-Rest steht, der zusammen mit dem Boratom, an das er gebunden ist, einen 5- oder 6-gliedrigen Ring bildet, der gegebenfalls durch einen oder mehrere C₁-C₄-AlkylReste substituiert ist,
m für die Zahl 2 steht,
p für die Zahl 1 steht;
(V) Boronate der Formel (III), in der
Q für OH, F, Cl, Br, I, C₁-C₄-Alkyl, C₆-C₁₀-Aryl, C₁-C₄-Alkoxy oder C₆-C₁₀-Aryloxy steht,
m für die Zahl 3 steht,
p für die Zahl 1 steht
und die negative Ladung des Boronatanions durch ein Kation ausgeglichen wird,
(VI) Triarylborane der Formel (III), in der
m für die Zahl 0 steht,
p für die Zahl 3 steht;
(VII) Tetraarylborate der Formel (IV), in der
m für die Zahl 0 steht,
p für die Zahl 4 steht
und die negative Ladung des Tetraarylboratanions durch ein Kation ausgeglichen wird,
in Gegenwart eines Katalysatorsystems bestehend aus einem Rutheniumkatalysator, einem Aktivierungsmittel, einem Oxidationsmittel und einem Metallsulfat umsetzt.

13. Verfahren zur Herstellung von Biphenylamiden der allgemeinen Formel (Ia) 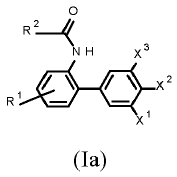 in welcher
R¹ für Wasserstoff, Hydroxy, Fluor, Chlor, C₁-C₄-Alkyl, C₁-C₄-Alkoxy, C₁-C₄-Alkylthio oder C₁-C₄-Halogenalkyl steht,
R² für C₁-C₄-Alkyl, C₆-C₁₀-Aryl oder C₆-C₁₀-Aryl-CH₂- steht
X¹ für Wasserstoff, C₁-C₃-Alkyl, C₁-C₄-Alkoxy, Fluor oder Chlor steht,
X² für Wasserstoff, C₁-C₃-Alkyl, C₁-C₄-Alkoxy, Fluor oder Chlor steht,
X³ für Wasserstoff, C₁-C₃-Alkyl, C₁-C₄-Alkoxy, Fluor oder Chlor steht,
**dadurch gekennzeichnet, dass** man
Anilide der Formel (II) 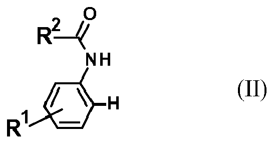 in welcher
R¹ und R² die oben angegebenen Bedeutungen haben,
mit einer Organoborverbindung der Formel (III) 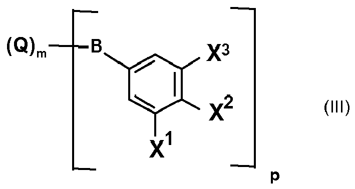 in welcher
X¹, X² und X³ die oben angegebenen Bedeutungen haben,
und die aus einer der folgenden Gruppen gewählt ist, bestehend aus
(I) Boronsäuren der Formel (III), in der
Q für eine Hydroxygruppe steht,
m für die Zahl 2 steht,
p für die Zahl 1 steht,
oder den Anhydriden, Dimeren oder Trimeren dieser Boronsäuren;
(II) Boronsäurederivaten der Formel (III), in der
Q für F, Cl, Br, I, C₁-C₄-Alkyl, C₆-C₁₀-Aryl, C₁-C₄-Alkoxy oder C₆-C₁₀-Aryloxy steht,
m für die Zahl 2 steht,
p für die Zahl 1 steht;
(III) Borinsäuren der Formel (III), in der
Q für OH, F, Cl, Br, I, C₁-C₄-Alkyl, C₆-C₁₀-Aryl, C₁-C₄-Alkoxy oder C₆-C₁₀-Aryloxy steht,
m für die Zahl 1 steht,
p für die Zahl 2 steht;
(IV) cyclische Boronsäureester der Formel (III), in der
Q für einen C₂-C₃-Alkyldioxy-Rest steht, der zusammen mit dem Boratom, an das er gebunden ist, einen 5- oder 6-gliedrigen Ring bildet, der gegebenfalls durch einen oder mehrere C₁-C₄-AlkylReste substituiert ist,
m für die Zahl 2 steht,
p für die Zahl 1 steht;
(V) Boronate der Formel (III), in der
Q für OH, F, Cl, Br, I, C₁-C₄-Alkyl, C₆-C₁₀-Aryl, C₁-C₄-Alkoxy oder C₆-C₁₀-Aryloxy steht,
m für die Zahl 3 steht,
p für die Zahl 1 steht
und die negative Ladung des Boronatanions durch ein Kation ausgeglichen wird,
(VI) Triarylborane der Formel (III), in der
m für die Zahl 0 steht,
p für die Zahl 3 steht;
(VII) Tetraarylborate der Formel (IV), in der
m für die Zahl 0 steht,
p für die Zahl 4 steht
und die negative Ladung des Tetraarylboratanions durch ein Kation ausgeglichen wird,
in Gegenwart eines Katalysatorsystems bestehend aus einem Rutheniumkatalysator, einem Aktivierungsmittel, einem Oxidationsmittel und einem Metalloxid umsetzt.
